# EUROPEAN PATENT APPLICATION

(11) **EP 2 664 367 A1**
(43) Date of publication of application: **20.11.2013**
(21) Application number: 12167980.7
(22) Date of filing: 15.05.2012
(51) Int. Cl.: B01D 29/085

(54) **Stackable cell strainer**

(71) Applicant: Miltenyi Biotec GmbH, 51429 Bergisch Gladbach (DE)
(72) Inventor: Poggel, Carsten Dr., 50859 Köln (DE); Kabaha, Eiad Dr., 53229 Bonn (DE); Adams, Timo, D-51519 Odental (DE); Stöters, Wolfgang, 45481 Mülheim/Ruhr (DE); Bosio, Andreas, 50939 Köln (DE)
(74) Representative: Kisters, Michael Marcus

(57) **Abstract**

The invention is directed to a cell strainer for separating particles from a cell suspension, comprising an upper portion with at least one filter area essential perpendicular to the direction of flow of the suspension and a lower portion adapted to fit into the openings of at least two tubes with different sized openings wherein the lower portion has a first section comprising shoulders or flanges having the diameter of the opening of a first tube and at least one second section comprising a recess for receiving the neck of a second tube, wherein the diameter of the first section is larger as the diameter of the second section.

## Description

The invention is directed to a filter system for removing particles above a certain diameter from a suspension containing single cells or cellular compartments wherein the outer shape of the filter system is adapted to fit to at least two laboratory vessels having different sized openings.

### Prior Art

In biological research or diagnosis in medicine, it is often required to dissociate a sample, like tissue into isolated cells. Tissue dissociation is often achieved by enzymatic digestions and/or mechanical dissection using fragmentation devices, for example as disclosed in EP1843852 B1. In case of dissociation, the single-cells are separated from undissociated tissue fragments by filtration.

Filter systems for biological research or diagnosis in medicine are long known. Gauze was initially used for separating isolated cells from bigger particles or tissue. As gauze absorbes the liquid of the sample in part, so called cell strainers comprising a mesh for filtration are used for this purpose. Cells strainers are available in several mesh sizes depending on the particles to be separated, for example on the size of the target cells.

Since the most common laboratory vessels for biological research or in medical diagnosis are so called tubes, i.e. cylinder-shaped vessels with a flip or screw cap, it is desired to perform the filtration into such tubes without the need of further handling of the sample. Tubes are available for several different volumes, having different sized openings. For researchers working with cells, especially 15ml and 50 ml are common tube formats.

Commercially available cell strainers for laboratory use are disposable, sterile packed and available for use with different vessels and applications. For example, EP 0593767 discloses a filter system which is inserted into a tube in a way that the filter flange is supported by the opening of the tube. In other words, the filter systems hangs in the tube, which means that this filter system will only work with the tube size they are designed for.

Since the filter disclosed in EP 0593767 hangs in the tube and has an outer diameter having about the inner diameter of the tube. Since essentially no space is left between the side walls of filter and tube, venting is impaired when the filter is filled with suspension. The result is a "flow stop", especially if a slightly viscous samples is processed and the filter needs to be lifted manually. The filter area is provided in a frame with a grip handle in order to handle the filter and preserve aseptic conditions without touching the sieve area. However, the grip handle is quite small and difficult to handle under the usual working conditions in a laboratory. Furthermore, the volume of the filter system above the sieve is too small to hold the volume of a standard tube, which makes refilling necessary if liquid doesn't run through the sieve immediately during pipetting.

Furthermore, filter systems designed for laboratory vessels with a small volume are usually small size objects with a small filter area. Small filter areas tend to clog and/or processing speed is rather low. On the other hand, handling of tubes with smaller diameter has clear advantages in reduced wash volumes, rack space, higher number of samples to be processed with a centrifuge and better pellet formation after centrifugation.

Accordingly there is a need for a filter system which provides a sufficient filter efficiency but is also compatible to small tube sizes.

### Object of the invention

Object of the invention was therefore to provide a cell strainer which has a high filter efficiency and is compatible with tubes of different opening size i.e. is fitting on at least the standard 15 ml and 50ml laboratory tubes.

This object is accomplished with a cell strainer for separating particles from a cell suspension, comprising an upper portion with at least one filter area essential perpendicular to the direction of flow of the suspension and a lower portion adapted to fit into the openings of at least two tubes with different sized openings wherein the lower portion has a first section comprising shoulders or flanges having the diameter of the opening of a first tube and at least one second section comprising a recess for receiving the neck of a second tube, wherein the diameter of the first section is larger than the diameter of the second section.

The at least one filter area located essential perpendicular to the direction of flow of the suspension may be located exactly perpendicular to the direction of flow or slightly deviated (0° - 15 °) to the direction of flow.

The cell strainer according to the invention can be used for separating particles from a cell suspension. Separating particles includes, for example, separating single cells from tissue which has been dissociated by mechanical (like grinding or fragmenting) or chemical means (like enzymatic digestion). In this case, target cells are separated from unwanted particles. Furthermore, separating particles includes the separation of cellular compartments like mitochondria, nuclei, ribosomes and vesicles obtained by desintegrating cells from intact cells or tissue. Also the cell strainer can be used itself for dissociation of tissue by pushing it (e.g. spleen) through the mesh of the cell strainer.

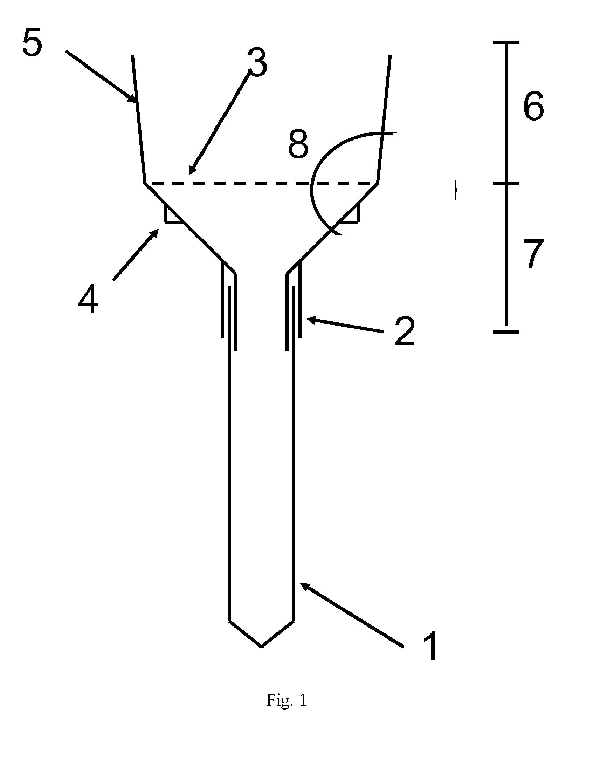

Fig. 1 shows a schematic side view of a cell strainer according to the invention: A 15 ml standard tube (1) is inserted into the recess (2) of the lower portion (7) of the cell strainer. The lower portion (7) comprises for resting on the opening of a 50 ml standard tube (not shown) shoulders or flanges (4). The upper portion (6) comprises the sieve area (3) and a reservoir volume (5).

The sieve area of cell strainer according to the invention is not incorporated in a frame but in the closed body of the upper portion. Therefore, the cell strainer can be easily taken out of the sterile package without touching the sieve, avoiding contamination of the sieve, and according to the invention does not need a grip or handle projecting from any part of the strainer. Tubes with such cell strainers installed can be stored in standard racks without difficulty.

In a first embodiment of the invention, the diameter of the filter area is larger than the inner diameter of the first and second section of the lower portion of the cell strainer. Of course, the inner diameter of the first and second section corresponds to the outer diameter of the tubes to be placed at the recess (2) and shoulders or flanges (4). It is possible to utilize filter areas with a smaller diameter than the inner diameter of the first and second section of the lower portion of the cell strainer. In this variant, the upper portion of the cell strainer is provided with more storage volume, but the filter surface area is reduced. For most applications, this variant of the invention is less preferred.

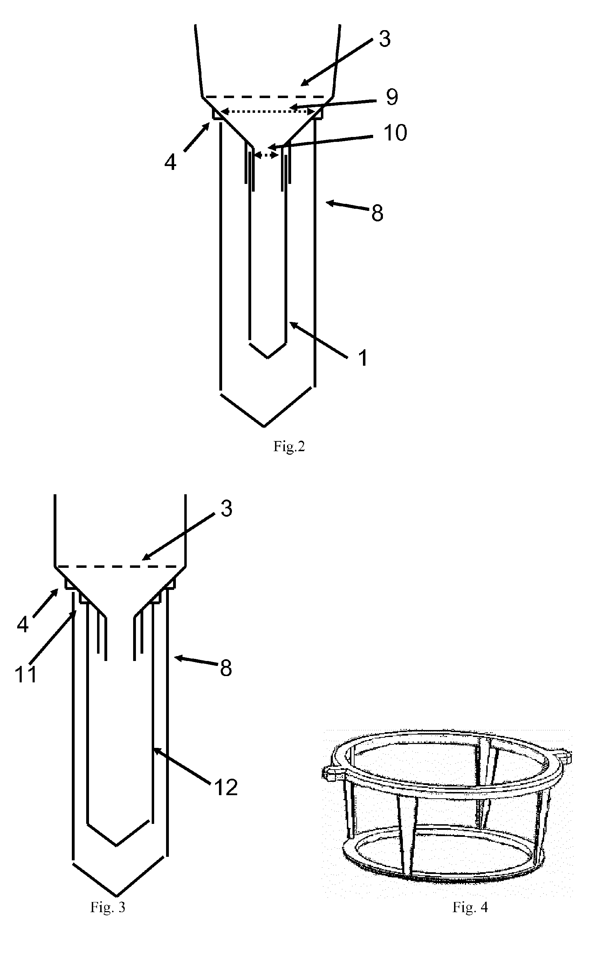

Fig.2 shows a side view of a cell strainer with a 15 ml standard tube (1) and a 50 ml standard tube (8) in place. The filter area (3) has a diameter larger than the inner diameter (9 and 10) of the first and second section i.e. at the recess (2) and shoulders or flanges (4). Preferably, the diameter of the filter area (3) is at least 1%, preferably 10 % to 25 % larger than the inner diameter (9) of the first and at least 25%, preferably 50% to 150% larger than the inner diameter (10) of the second section.

In another embodiment, the upper portion of the cell strainer has an external diameter substantially equal to the lid of a standard 50 ml tube.

The upper portion of the cell strainer can be substantially cylinder or cone- shaped. The lower portion is substantially shaped as a cone or can be provided with at least two cone-shaped sections with different external diameters. Fig. 3 shows a cell strainer with a cylindrical upper portion.

Regardless of their shape, the upper and lower portions of the cell strainer share an angle with each other. Especially if using the cell strainer with a 15 ml standard tube, the angle should not be too small to prevent clogging. If the angle is too large, the extensions into the tubes would be too big and the overall size of the cell strainer would prevent stacking of at least two cell strainers. The angle between upper and lower portion of the cell strainer is preferable between 105 and 165° as shown in (8) in Fig. 1.

In another embodiment of the invention, the cell strainer is provided with an upper portion having an internal diameter and internal space to allow stacking of at least two cell strainers. The upper portion of the cell strainer has preferable a volume of at least 10 ml, especially 15 to 30 ml (5 in Fig 1).

Stackable cell strainers can be used for sequential or fractionated filtration, i.e. the cell suspension is first filtered through a cell strainer having a large mesh size into a second cell strainer having a smaller mesh size and if necessary into further strainers having an even smaller mesh size. Coarse particles can be removed from a cell suspension without clogging the filter and thus reducing the processing time.

The cell strainer may comprise two, three, four of five sections of the lower portion with different external diameters with shoulders or flanges to receive openings of tubes with different internal diameter. Fig. 3 shows a cell strainer with two sets of shoulders or flanges (4 and 11) for tubes (8) and (12). Particular, cell strainers comprise two sections of the lower portion with different external diameters fitting into tubes having 15 ml and 50 ml volume. Common tubes having 15 ml and 50 ml volume can be obtained, for example, as BD Falcons or Corning Centrifuge Tubes.

The shoulders or flanges on the lower portion of the cell strainer are preferable arranged in a way to enable a tilt-free stacking of the cell strainers. The term "shoulders or flanges" used in this patent application is intended to encompass any structure like edges or rims formed on the lower portion of the cell strainer to provide suitable mechanical support for a cell strainer on or into the opening of a tube-like vessel. A skilled artesian is aware of structures which enable the lower portion of the cell strainer to be stacked on a tube in a tilt-free manner.

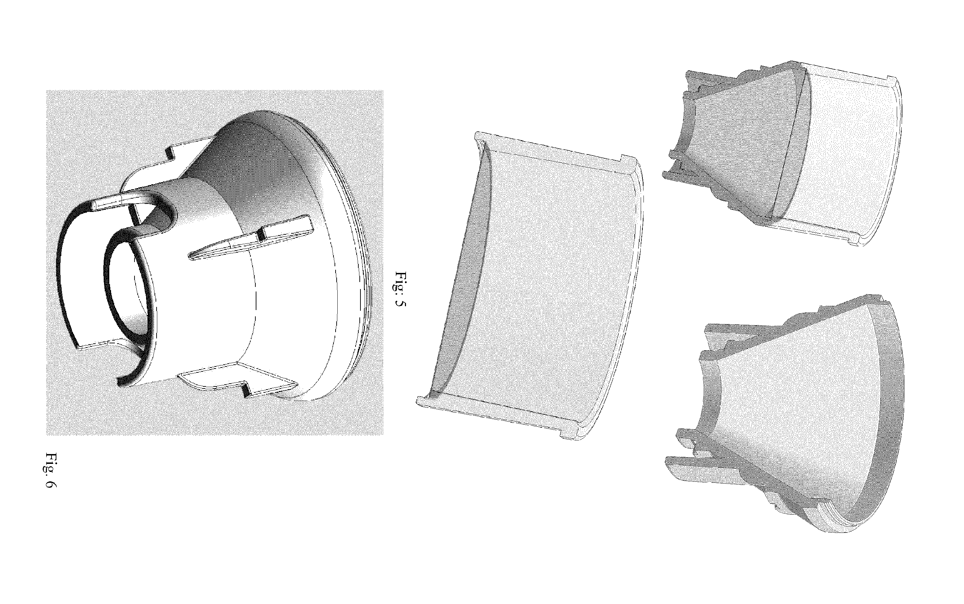

In a further embodiment of the invention, the cell strainer is provided with shoulders or flanges having at least one recess for venting. The shoulders or flanges may comprise between 1 and 25 recesses for venting. Fig. 5 shows a cell strainer with a plurality of recesses in a shoulder/rim-like support structure on its lower portion. In a variant of the invention shown in Fig. 6, the cell strainer comprises rather small flanges with broad venting area between the flanges. In such case, the cell strainer may comprise 3 to 10 flanges with venting areas between the flanges acting the same way as recesses in a shoulder/rim/edge-like structure.

It was found that filtration area oriented parallel to the direction of flow does not contribute to filter performance or capacity as much as filtration area oriented perpendicular to the direction of flow. For example, by enlarging the filtration area oriented perpendicular to the direction of flow by 35 %, roughly the same filtration throughput is reached as by enlarging the filtration area oriented parallel to the direction of flow by 170 %. Accordingly, the cell strainer is preferable provided with only one filter area perpendicular to the direction of flow of the cell suspension. Therefore, it is more efficient to have a filtration area perpendicular to the flow direction which diameter is bigger than the tube than to have the feature of hanging the filter into the tube.

For large amounts of cell suspension to be filtrated, it might be necessary to provide more filtration area as perpendicular to the direction of flow available. For such cases the cell strainer can be provided with an upper portion comprising at least one filter area perpendicular to the direction of flow of the cell suspension and at least one filter area in the direction of flow of the cell suspension.

At least one filter area of the cell strainer can be provided in a frame located in the upper portion of the cell strainer. The frame can comprise at least one filtration area perpendicular to the direction of flow and/or at least one filter area in the direction of flow of the cell suspension. Fig. 4 shows by way of example a filter frame to be inserted into the cell strainer.

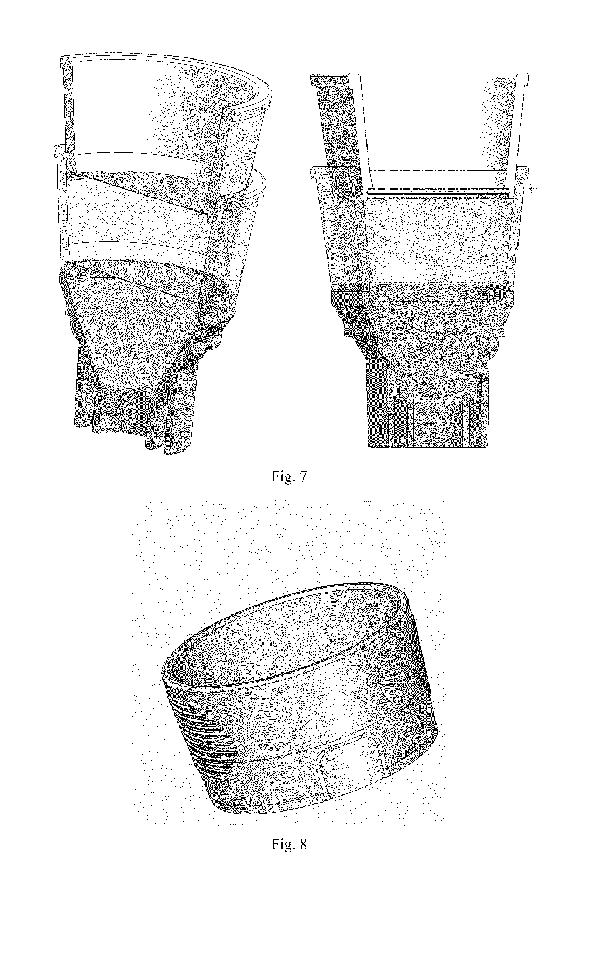

The upper and lower portion of the cell strainer according to the invention can be either integrally formed or formed as separate bodies (see Fig. 5). In the first case, the cell strainer consists of one piece. If the cell strainer comprises upper and lower portion which can be separated, the upper portion comprising the filter area may be disconnected from the lower portion and stacked on a second cell strainer. Fig. 7 shows stacked cell strainers of this embodiment.

For better handling, especially when the user of the cell strainer is wearing gloves, the upper portion of the cell strainer can be provided with fine ripples of grooves which may be orientated in or perpendicular to the direction of flow. Fig. 8 shows an upper portion of the cell strainer separated from the lower portion equipped with grooves.

In another embodiment of the invention, the outer walls of the recess of the second section of the lower portion are provided with openings. The openings enable the user of the cell strainer to observe the fill level of an installed smaller tube. In addition, it is preferable that the inner walls of the recess of the second section of the lower portion are shorter than the outer walls. Both embodiments are depicted in Fig. 6.

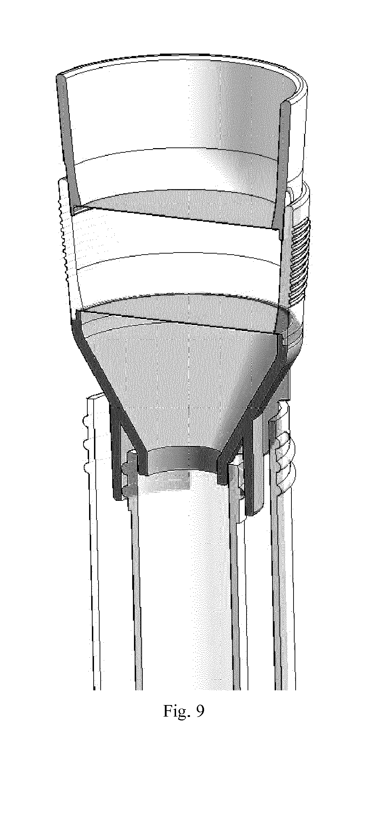

Fig. 9 shows a side view of stacked cell strainers with a combination of the following features of the invention:
- The diameter of the filter area is larger than the diameter of the first and second section.
- Small flanges with broad venting area between the flanges
- upper portion is provided with groove
- lower portion is substantially shaped as a cone sharing an angle of 105-165° with the upper portion
- the recess of the second section is shaped to receive the neck of a standard 15 ml tube.
- upper and lower portion of the cell strainer are separate bodies.
- the outer walls of the recess of the second section of the lower portion is provided with openings to allow controlling the liquid level in the second tube
- the inner walls of the recess of the second section of the lower portion are shorter than the outer walls.

Fig. 9 shows for better understanding of the invention two installed tubes which is not the case in normal use of the cell strainer. It should be noted that not all features of the invention as shown in Fig.9 need to be implemented simultaneously on a cell strainer.

The cell strainer may be produced from various materials, preferably from plastics like, for example, polystyrene (PS), polyvinylchloride (PVC), polycarbonate, glass, polyacrylate, polyacrylamide, polymethylmethacrylate (PMMA), polyethylene terephthalate (PET), polytetrafluorethylen (PTFE), thermoplastic polyurethane (TPU), silicone, polyethylene (PE) polypropylene (PP), polyvinyl alcohol (PVA) or compositions comprising one or more of the above mentioned materials.

The cell strainer according to the invention may comprise filter areas with mesh sizes between 10 and 500 µm, for example 10, 20, 30, 40, 50, 60, 70, 80, 90, 100, 150, 200 or 500 µm. The filter may be produced from plastics like polyamide (PA), polystyrene (PS), polyvinylchloride (PVC), polycarbonate, glass, polyethylene terephthalate (PET), polytetrafluorethylen (PTFE), silicone, poly ethylene (PE), poly propylene (PP) and/or polyvinyl alcohol (PVA), polyethersulfon (PES).

### Examples

The efficiency of a cell strainer according to the invention (as shown in Fig. 1-3, 5 and 6) and the prior art (EP 0593767, similar to Fig.4) were compared. Both cell strainers were fixed in a stand a few mm above a 50mL FALCON tube so that the filtrate drops into the tube without the risk of a flow stop which sometimes appears with viscous cell suspensions. A mouse liver cell suspension was created by dissociation of mouse liver in 10 mL PEB with program B on the gentleMACS dissociator. The cell suspension was diluted with 90mL PEB. 9mL of the diluted cell suspension was given on each Cell Strainer. The flow-through was collected and the volume was measured.

The cell strainer according to the invention had a bottom filter area of 346mm², whereas the cell strainer according to the prior art (as shown in Fig. 4) had a side filter area of 595mm² and a bottom filter area of 346 mm² (total filter area: 941 mm²).

For better comparison, the side walls of the cell strainer of the prior art were in quarters sealed.

For an efficient cell strainer, it would be expected that the flow-through volume increases proportional to the filter area. As can be seen from the following table, although the filter area of the cell strainer according to the prior art has 172% of the filter area of the cell strainer according to the invention, its flow-through is increased only by 55%. This is caused by a lower filtration efficiency of the side wall the filter area in comparison to the bottom filter area.

| Filter | volume of flow-through in mL | Average | Filter area in mm² | µl/mm² | % increase of filtrate volume |
|---|---|---|---|---|---|
| prior art (no sealing) | 6.5 | 6.2 | 941 | 6.6 | 55 |
| | 6.5 | | | | |
| | 5.5 | | | | |
| prior art (one quarter sealed) | 5.3 | 5.7 | 792 | 7.2 | 8 |
| | 6.1 | | | | |
| | 5.7 | | | | |
| prior art (2 quarters sealed) | 5.1 | 5.3 | 644 | 8.2 | 14 |
| | 5.3 | | | | |
| | 5.5 | | | | |
| prior art (3 quarters sealed) | 4.5 | 4.8 | 495 | 9.6 | 23 |
| | 4.7 | | | | |
| | 5.1 | | | | |
| According to the invention | 4.4 | 4.0 | 346 | 11.5 | - |
| | 3.6 | | | | |
| | 3.9 | | | | |

In another experiment it was shown that an increase of the filter area according to the invention by 36% (475mm²) resulted in higher flow-through volumes than the cell strainer of prior art (941 mm²).

Based on the same filter area, the cell strainer according to the invention is more efficient than those of the prior art. For most of the filtration problems, the filtration capacity of the cell strainer according to the invention is sufficient.

Furthermore, due to its shape as defined in the present claims, the cell strainer according to the invention could be placed securely on both the standard 15 ml and 50ml laboratory tubes, whereas the cell strainer of the prior art fits only into the standard 50ml laboratory tubes or needs a separate stand.

## Claims

1. Cell strainer for separating particles from a cell suspension, comprising an upper portion with at least one filter area essential perpendicular to the direction of flow of the suspension and a lower portion adapted to fit into the openings of at least two tubes with different sized openings **characterized in that** the lower portion has a first section comprising shoulders or flanges having the diameter of the opening of a first tube and at least one second section comprising a recess for receiving the neck of a second tube, wherein the diameter of the first section is larger than the diameter of the second section.

2. Cell strainer according to claim 1 **characterized in that** the diameter of the filter area is larger than the diameter of the first and second section.

3. Cell strainer according to claim 1 or 2 **characterized in that** the shoulders or flanges are provided with at least one recess for venting.

4. Cell strainer according to any of the claims 1 to 3, **characterized in that** the upper portion is substantially shaped as cylinder and the lower portion is substantially shaped as a cone sharing an angle of 105-165°.

5. Cell strainer according to any of the claims 1 to 4, **characterized in that** the upper portion is substantially shaped as cylinder and the lower portion has at least two cone-shaped sections with different external diameter.

6. Cell strainer according to any of the claims 1 to 5, **characterized in that** the upper portion comprises at least one filter area perpendicular to the direction of flow of the cell suspension and at least one filter area in the direction of flow of the cell suspension.

7. Cell strainer according to any of the claims 1 to 6, **characterized in that** the outer diameter of the shoulders or flanges of the first section is substantially equal to the inner diameter of a standard 50 ml tube.

8. Cell strainer according to any of the claims 1 to 7, **characterized in that** the recess of the second section is shaped to receive the neck of a standard 15 ml tube.

9. Cell strainer according to any of the claims 1 to 8, **characterized in that** the upper portion has a third section having an outer diameter substantially equal to the inner diameter of the upper portion in order to allow stacking of cell strainers.

10. Cell strainer according to any of the claims 1 to 9, **characterized in that** the upper and lower portions are integrally formed.

11. Cell strainer according to any of the claims 1 to 9, **characterized in that** the upper and lower portions are separate bodies.

12. Cell strainer according to any of the claims 1 to 11 **characterized in that** the outer walls of the recess of the second section of the lower portion is provided with openings.

13. Cell strainer according to any of the claims 1 to 12 **characterized in that** the inner walls of the recess of the second section of the lower portion are shorter than the outer walls to minimize extension of the cell strainer into the second tube.
